# EUROPÄISCHE PATENTSCHRIFT

(11) **EP 1 307 498 B1**
(45) Veröffentlichungstag und Bekanntmachung des Hinweises auf die Patenterteilung: **08.02.2006**
(21) Anmeldenummer: 01982205.5
(22) Anmeldetag: 01.08.2001
(51) Int. Cl.: C08G 61/00, C09K 11/06, H05B 33/14, H01L 51/50, B41M 5/24

(54) **STRUKTURIERBARE MATERIALIEN, VERFAHREN ZU DEREN HERSTELLUNG UND DEREN VERWENDUNG**
MATERIALS THAT CAN BE STRUCTURED, METHOD FOR PRODUCING THE SAME AND THEIR USE
MATERIAUX STRUCTURABLES, LEUR PROCEDE DE FABRICATION ET LEUR UTILISATION

(30) Priorität: 01.08.2000 DE 10037391
(43) Veröffentlichungstag der Anmeldung: 07.05.2003
(73) Patentinhaber: Covion Organic Semiconductors GmbH, 65926 Frankfurt (DE)
(72) Erfinder: MEERHOLZ, Klaus, 81369 München (DE); BAYERL, Michael, 85586 Poing (DE); BIELEFELDT, Florian, 4052 Basel (CH); BRAIG, Thomas, 80809 München (DE); GROSS, Markus, 82054 Sauerlach (DE); MÜLLER, David, 80805 München (DE); NUYKEN, Oskar, 81927 München (DE); SPREITZER, Hubert, 68519 Viernheim (DE)
(74) Vertreter: Luderschmidt, Schüler & Partner GbR
(86) Internationale Anmeldenummer: PCT/EP2001/009176
(87) Internationale Veröffentlichungsnummer: WO 2002/010129

(56) Entgegenhaltungen:
- WO-A-96/19792
- WO-A-98/53510
- T.BRAIG, D.C.MÜLLER, M.GROSS, K.MEERHOLZ, O.NUYKEN: "Crosslinkable hole-transporting polymers by palladium catalyzed C-N-coupling reaction" MACROMOL. RAPID COMMUN., Bd. 21, Nr. 9, Juni 2000 (2000-06), Seiten 583-589, XP002193425
- M.S.BAYERL, T.BRAIG, O.NUYKEN, D.C.MÜLLER, M. GROSS, K.MEERHOLZ: "Crosslikable hole-transport materials for preparation of multilayer organic light emitting devices by spin-coating" MACROMOL. RAPID COMMUN., Bd. 20, 1999, Seiten 224-228, XP002193426

## Beschreibung

Seit wenigen Jahren existiert ein stark wachsender Bedarf an organischen Materialien, die für den Einsatz in Elektronik-Anwendungen benötigt werden. Typische Anwendungen sind solche in organischen (OLED) oder polymeren Leuchtdioden (PLED) (vgl. z. B. EP-A-0 676 461, WO 98/27136), organischen Solarzellen (vgl. z. B. WO 98/48433, WO 94/05045), organischen Lasern (z. B. WO 98/03566) und auch in organischen Schaltungkreisen (ICs) (z. B. WO 95/31833, WO 99/10939).

Die Verwendung von Materialien ist den o. g. Anmeldungen und Patenten und den darin zitierten Literaturstellen beschrieben und wird hier nicht weiter vertieft.

Für die Verwendung in den o. g. Anwendungsgebieten kommen sowohl niedermolekulare, als auch polymere Verbindungen zum Einsatz. Hierbei kommen für die organischen bzw. polymeren Leuchtdioden und Laseranwendungen sowohl aktive Materialien (d. h. lichtemittierende Substanzen), als auch Materialien für weitere Hilfsschichten, d. h. zum Beispiel Ladungstransportschichten mit bestimmten optischen Eigenschaften in Frage.

Für die Verwendung in den Anwendungsgebieten Solarzellen und organische Schaltelemente sind bevorzugt organische Halbleiter bzw. Isolatoren, und damit i. d. R. Ladungstransportmaterialien mit unterschiedlichen Transporteigenschaften, gefordert.

Bei all diesen Anwendungen werden typischerweise im Bereich der hochmolekularen Verbindungen konjugierte oder zumindest teilkonjugierte Polymere eingesetzt. Typische Vertreter konjugierter Polymerer sind Poly-(p-phenylen-vinylen) [PPV] oder Poly-p-phenylen [PPP]. Wenn die PPP-Struktur überwiegend aus Fluoren-Bausteinen aufgebaut ist, werden diese Materialien auch als Polyfluorene bezeichnet. Wenn die PPP-Struktur überwiegend Spiro-9,9'-bifluoren-Einheiten enthält spricht man auch von Poly-Spiros.

Teilkonjugierte Polymere im Sinne dieser Erfindung sollen solche Substanzen sein, die entweder in der Hauptkette hauptsächlich konjugierte Segmente aufweisen, die von nichtkonjugierten Segmenten unterbrochen sind, bzw. solche, die längere konjugierte Segmente in der Seitenkette besitzen.

Andererseits sind auch niedermolekulare Verbindungen in einigen der o. g. Anwendungsgebieten bereits erfolgreich im Einsatz. Unter den niedermolekularen Verbindungen haben solche mit aromatischen π-Systemen (Aromaten) besondere Bedeutung erlangt. Neben 2-dimensionalen Aromaten wie z.B. Triphenylen-Derivaten (DE-A-4422332), haben insbesondere Aromaten mit 3-dimensionater räumlich ausgedehnter Struktur sich als vorteilhaft erwiesen. Typische Vertreter sind Verbindungen , die auf Spirobifluoren (EP-A-676461), Triptycen oder Iptycen (DE-A-19744792) basieren.

Für alle diese Anwendungen gibt es zwar bereits verschiedenartige Substanzen, die Verwendung finden, die Entwicklung derartiger Verbindungen ist jedoch keinesfalls als abgeschlossen zu betrachten.
So ist zum einen ein starker Druck gegeben, die operative Lebensdauer der Verbindungen in den entsprechenden Anwendungen zu erhöhen, zum anderen ist bei entsprechenden Anwendungen auch die Strukturierung ein nicht gelöstes Problem.

Die Strukturierung ist je nach Einsatzgebiet ein sehr wichtiges Kriterium: Bei Displays (basierend auf OLED oder PLED Technologie) müssen beispielsweise die einzelnen Bildpunkte (Pixel) geschaffen werden. Ein ähnliches Problem stellt sich natürlich auch bei der Erzeugung organischer Schaltkreise und teilweise auch bei der Strukturierung von organischen Solarzellen-Pannels bzw. Laser-Arrays. Üblicherweise werden diese Strukturierungen bei den "Zuleitungen", d. h. beispielsweise bei den Elektroden durchgeführt. Dies kann z. B. durch Schattenmasken nach Art einer Schablone erfolgen; für die industrielle Massenfertigung kann dies jedoch deutliche Nachteile ergeben: Die Masken sind nach ein- oder mehrmaliger Benutzung unbrauchbar durch Belagbildung und müssen aufwendig regeneriert werden.

Eine weitere Möglichkeit der Strukturierung besteht auch darin, die aktive Schicht (hier: entweder die lichtemittierende Schicht in OLEDs/PLEDs und Lasern bzw. Ladungstransportschichten in allen Anwendungen) direkt strukturiert aufzubringen. Da dies erhebliche Probleme bereitet ist alleine schon aus den Dimensionen verständlich: es müssen Strukturen im Bereich von einigen 10 µm bei Schichtdicken im Bereich von ca. 100 nm bis zu wenigen µm geschaffen werden. Hierzu geeignet könnten eventuell Druckverfahren (wie Off-Set-Drucken, Tintenstrahl-Drucken, o. ä. Techniken) sein, allerdings ist noch kein derartiges Verfahren wirklich produktionstauglich. Ebenfalls wird hier (im Bereich der OLEDs) die oben bereits für die Elektroden geschilderte Maskentechnologie verwendet. Diese bringt hier jedoch deutlich die oben geschilderten Probleme der Belagbildung mit sich.

Aus Polym. Materials, Science and Engineering 80, 122 (1999) sind nun mit oxetangruppen funktionalisierte N,N,N',N'-Tetraphenylbenzidine bekannt, die photoinduziert vemetzbar sind. Die vorstehend genannte Klasse von Verbindungen werden als strukturierbare Lochleiter in organischen Leuchtdioden (OLED) eingesetzt, so daß strukturierte OLED erzeugt werden können.

Aufgabe der vorliegenden Erfindung war es daher, strukturierbare Materialien bereitzustellen, die zur Verwendung in strukturierten Vorrichtungen, wie OLEDs, PLEDs, organischen Lasern, organischen Schaltelementen, und organischen Solarzellen geeignet sind, wobei das Eigenschaftsprofil dieser Vorrichtungen zumindest beibehalten ist.

Es wurde nun überraschend gefunden, daß organische Materialien in den oben aufgezählten Anwendungen strukturiert werden können, wenn sie mindestens eine zur Vernetzung befähigte Oxetan-Gruppe enthalten, deren Vernetzungsreaktion gezielt ausgelöst und gesteuert werden kann. Dies kann unter geeigneten Bedingungen so durchgeführt werden, daß keine Beeinträchtigung der ansonsten vorhandenen Vorrichtungs-Charakteristik eintritt, jedoch damit die Strukturierung deutlich vereinfacht wird bzw. überhaupt erst möglich gemacht wird.

Gegenstand der Erfindung sind daher niedermolekulare oder polymere organische Materialien, die in den oben genannten elektronischen Anwendungen Verwendung finden, bei denen mindestens ein H-Atom durch eine Gruppe der Formel (A) ersetzt ist, 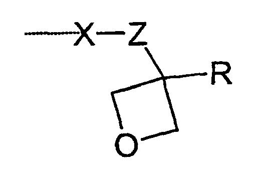 (A) worin
- R: eine geradkettige, verzweigte oder cyclische Alkyl-, Alkoxyalkyl, Alkoxy- oder Thioalkoxy-Gruppe mit 1 bis 20 C-Atomen, C4-C18-Aryl, C2-C10-Alkenyl bedeuten bei denen ein oder mehrere Wasserstoffe durch Halogen, wie Cl und F, oder CN ersetzt sein können und ein oder mehrere nicht benachbarte C-Atome durch -O-, -S-, -CO-, -COO-, -O-CO-, ersetzt sein können,
- Z: für -O-, -S-, -CO-, -COO-, -O-CO- oder eine bivalente Gruppe -(CR₁R₂)ₙ- bei der R1 und R2 unabhängig voneinander Wasserstoff , eine geradkettige, verzweigte oder cyclische Alkyl-, Alkoxy-, Alkoxyalkyl oder Thioalkoxy-Gruppe mit 1 bis 20 C-Atomen, C4-C18-Aryl, C2-C10-Alkenyl bedeuten bei denen ein oder mehrere Wasserstoffe durch Halogen, wie Cl und F oder CN ersetzt sein können und ein oder mehrere nicht benachbarte C-Atome durch -O-, -S-, -CO-, -COO-, -O-CO- ersetzt sein können,
- X: eine bivalente Gruppe-(CR₁R₂)ₙ- bei der R1 und R2 unabhängig voneinander Wasserstoff, eine geradkettige, verzweigte oder cyclische Alkyl-, Alkoxy-, Alkoxyalkyl oder Thioalkoxy-Gruppe mit 1 bis 20 C-Atomen, C4-C18-Aryl, C2-C₁₀-Alkenyl bedeuten bei denen ein oder mehrere Wasserstoffe durch Halogen, wie Cl und F oder CN ersetzt sein können, und
- n: eine ganze Zahl zwischen 1 und 20, vorzugsweise zwischen 3 und 10, insbesondere 3 oder 6, bedeutet,

mit der Maßgabe, daß die Anzahl dieser Gruppen A durch die maximal verfügbaren, d.h. substituierbaren H-Atome begrenzt ist.

Bei den erfindungsgemäßen Elektronik-Materialien handelt es sich um Elektrolumineszenz- bzw. Lasermaterialien wie
A) Homo- oder Copolymere auf Basis von PPV oder Poly-Fluorenen oder Poly-Spiro
B) Niedermolekulare Verbindungen mit 3-dimensionaler Spirobifluoren-Struktur,
C) Niedermolekulare Verbindungen mit 3-dimensionale Triptycen-Struktur
D) Niedermolekulare Verbindungen mit 2-dimensionaler Triphenylen-Struktur
E) Derivate des Perylen-tetra-carbonsäure-diimids
F) Derivate des Chinaciridons
G) organische Lanthanoid-Komplexe
H) Derivate des Aluminium-tris-chinoxalinats
I) Oxadiazol-Derivate
   oder um Lochleiter wie
J) Polystyrole, Polyacrylate, Polyamide, Polyester, die Derivate des Tetra-arylbenzidins in der Seitenkette tragen,
K) Niedermolekulare Verbindungen mit 2-dimensionaler Triphenylen-Struktur oder um Elektronenleiter wie
L) Derivate des Aluminium-tris-chinoxalinats
M) Oxadiazol-Derivate

Der Oxetan-Gehalt wird definiert durch das molare Verhältnis von Oxetan-Ringen bezogen auf die Gesamtheit von organischen Ringen, d.h. einschließlich der Oxetan-Ringe in der jeweiligen Struktur. Diese läßt sich im allgemeinen durch analytische Methoden bestimmen. Eine der bevorzugten Methoden neben der IR-Spektroskopie ist die Kernspin-Resonanz-Spektroskopie (NMR).

Ringe im Sinne der Erfindung sind cyclische Strukturelemente gebildet aus mindestens drei Ringatomen mit der Maßgabe, daß mindestens zwei C-Atome enthalten sind (The Ring Index, Patterson and Capell, Reinhold Publishing Company, 1940 und Handbook of Chemistry and Physics, 62^{nd} ed.1981, C-48).

Der Oxetan-Gehalt kann in weiten Bereichen von 0,01 bis 0,6 variiert werden. Im unteren Bereich werden niedrige Vemetzungsgrade erzielt, die relativ weiche, gummi-elastische bis gelartige Schichten ergeben. Im oberen werden hohe Vernetzungsdichten erreicht mit duroplast-artigen Eigenschaften, wie z.B. Bakelite.

A1) Die Homo- und Copolymere des PPVs enthalten ein oder mehrere Struktureinheiten der Formel (B), wobei mindestens ein H-Atom im Polymer durch einen Substituenten gemäß Formel (A) ersetzt sind.

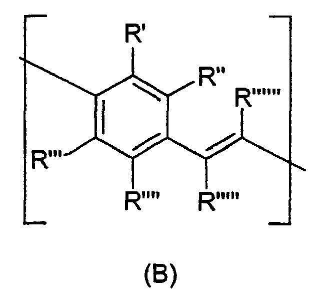

Die Substituenten R' bis R""" sind dabei gleich oder verschieden H, CN, F, Cl oder eine geradkettige, verzweigte oder cyclische Alkyl- oder Alkoxygruppe mit 1 bis 20 C-Atomen, wobei ein oder mehrere nicht benachbarte CH₂-Gruppen durch -O-, -S-, -CO-, -COO-, -O-CO-, -NR¹-, -(NR²R³)⁺-A⁻, oder -CONR⁴⁻ersetzt sein können und wobei ein oder mehrere H-Atome durch F ersetzt sein können, oder eine Arylgruppe mit 4 bis 14 C-Atome, die durch einen oder mehrere, nicht aromatische Reste R' substituiert sein kann.
- R1,R2,R3,R4: gleich oder verschieden aliphatische oder aromatische Kohlenwasserstoffreste mit 1 bis 20 C-Atomen oder auch H.
- A-:: ein einfach geladenes Anion oder dessen Äquivalent;

Bevorzugt sind hierbei PPVs gemäß WO 98/27136, die in Formel (C) wiedergegeben sind. 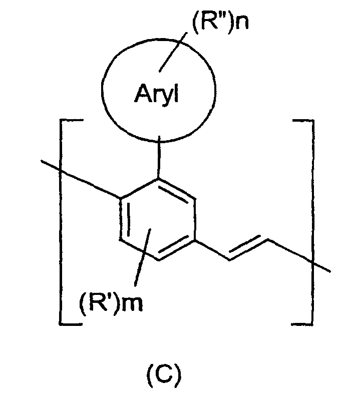
wobei die Symbole und Indizes folgende Bedeutungen haben:
- Aryl:: eine Arylgruppe mit 4 bis 14 C-Atomen;
- R', R":: gleich oder verschieden eine geradkettige oder verzweigte oder cyclische Alkyl- oder Alkoxygruppe mit 1 bis 20 C-Atomen, wobei ein oder mehrere nicht benachbarte CH₂-Gruppen durch -O-, -S-, -CO-, -COO-, -O-CO-, -NR¹-, -(NR²R³)⁺-A-, oder -CONR⁴- ersetzt sein können und wobei ein oder mehrere H-Atome durch F ersetzt sein können, CN, F, Cl oder eine Arylgruppe mit 4 bis 14 C-Atome, die durch einen oder mehrere, nicht aromatische Reste R' substituiert sein kann;
- R¹,R²,R³,R⁴: gleich oder verschieden aliphatische oder aromatische Kohlenwasserstoffreste mit 1 bis 20 C-Atomen oder auch H.
- A⁻:: ein einfach geladenes Anion oder dessen Äquivalent;
- m:: 0, 1 oder 2;
- n:: 1, 2, 3, 4 oder 5.

Besonders bevorzugt sind Polymere, bestehend hauptsächlich aus Wiederholeinheiten der Formel (C).

Ganz besonders bevorzugt sind weiterhin auch Copolymere, bestehend im wesentlichen aus, besonders bevorzugt bestehend aus Wiederholeinheiten der Formel (I) und weiteren Wiederholeinheiten, welche bevorzugt ebenso Poly(arylenvinylen)-Strukturen, besonders bevorzugt 2,5-Dialkoxy-1,4-phenylenvinylenstrukturen enthalten, wobei die Alkoxygruppen vorzugsweise geradkettig oder verzweigt sind und 1 bis 22 C-Atome enthalten.

Copolymere im Sinne der Erfindung umfassen statistische, alternierende, reguläre sowie blockartige Strukturen.

Ebenso bevorzugt sind Polymere, enthaltend Wiederholeinheiten der Formel (C), bei denen die Symbole und Indizes folgende Bedeutungen haben:
- Aryl: ist Phenyl, 1- bzw. 2-Naphthyl, 1-, 2- bzw. 9-Anthracenyl, 2-, 3- bzw. 4-Pyridinyl, 2-, 4- bzw. 5-Pyrimidinyl, 2-Pyrazinyl, 3- bzw. 4-Pyridazinyl, 2-, 3-, 4-, 5-, 6-, 7- bzw. 8-Chinolin, 2- bzw. 3-Thiophenyl, 2- bzw. 3-Pyrrolyl, 2- bzw. 3-Furanyl und 2-(1,3,4-Oxadiazol)yl;
- R': ist gleich oder verschieden, CN, F, Cl, CF₃ oder eine geradkettige oder verzweigte Alkoxygruppe mit 1 bis 12 C-Atomen;
- R": ist gleich oder verschieden eine geradkettige oder verzweigte Alkyl- oder Alkoxygruppe mit 1 bis 12 C-Atomen;
- n: ist 0, 1, 2 oder 3, besonders bevorzugt 0, 1 oder 2.

Die Herstellung derartiger Polymere ist in WO 98/27136 eingehend beschrieben. Die Herstellung entsprechender erfindungsgemäßer Polymere kann durch das einpolymerisieren, entsprechender Monomere, die die Oxetangruppierung der Formel (A) tragen, erfolgen.

A2) Die Homo- und Copolymere des Polyfluorene enthalten ein oder mehrere Struktureinheiten der Formel (D), wobei mindestens ein H-Atom im Polymer durch einen Substituenten gemäß Formel (A) ersetzt sind.

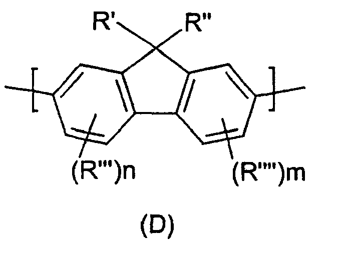

Die Substituenten R' bis R"" sind dabei gleich oder verschieden H, CN, F, Cl oder eine geradkettige, verzweigte oder cyclische Alkyl- oder Alkoxygruppe mit 1 bis 20 C-Atomen, wobei ein oder mehrere nicht benachbarte CH₂-Gruppen durch -O-, -S-, - CO-, -COO-, -O-CO-, -NR¹-, -(NR²R³)-A⁻, oder -CONR⁴- ersetzt sein können und wobei ein oder mehrere H-Atome durch F ersetzt sein können, oder eine Arylgruppe mit 4 bis 14 C-Atome, die durch einen oder mehrere, nicht aromatische Reste R' substituiert sein kann.
- R¹,R²,R³,R⁴: gleich oder verschieden aliphatische oder aromatische Kohlenwasserstoffreste mit 1 bis 20 C-Atomen oder auch H.
- A⁻:: ein einfach geladenes Anion oder dessen Äquivalent;
- n, m:: 0, 1, 2, oder 3, bevorzugt 0 oder 1.

A2.1) Bevorzugt sind dabei Strukturen gemäß DE-A-19846767 die im folgenden aufgeführt sind. Diese Polymere enthalten neben Struktureinheiten gemäß Formel (E1) 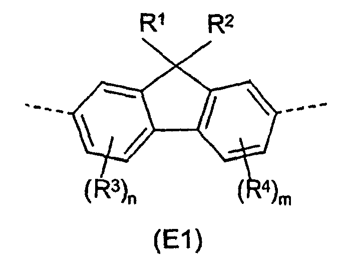
worin
- R¹, R²: gleich oder verschieden Wasserstoff, C₁-C₂₂-Alkyl, C₂-C₂₀-Heteroaryl, C₅-C₂₀₋Aryl, F, Cl, CN; wobei die vorstehend genannten Alkylreste verzweigt oder unverzweigt sein können oder auch Cycloalkyle darstellen, und einzelne, nicht benachbarte CH₂-Gruppen des Alkyrestes durch O, S, C=O, COO, N-R⁵ oder auch C₂-C₁₀-Aryle bzw. Heteroaryle ersetzt sein können, wobei die vorstehend genannten Aryle/Heteroaryle mit einem oder mehreren nichtaromatischen Substituenten R³ substituiert sein können. Bevorzugt sind Verbindungen, bei denen R¹ und R² beide gleich und dabei ungleich Wasserstoff oder Chlor sind; weiterhin bevorzugt sind Verbindungen, bei denen R¹ und R² voneinander verschieden und auch von Wasserstoff verschieden sind;
- R³, R⁴: gleich oder verschieden H, C₁-C₂₂-Alkyl, C₂-C₂₀-Heteroaryl, C₅-C₂₀-Aryl, F, Cl, CN, SO₃R⁵, NR⁵R⁶; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder C₂-C₁₀-Aryle bzw. Heteroaryle ersetzt sein können, wobei die vorstehend genannten Aryle/Heteroaryle mit einem oder mehreren nichtaromatischen Substituenten R³ substituiert sein können,
- R⁵, R⁶: gleich oder verschieden H, C₁-C₂₂-Alkyl, C₂-C₂₀-Heteroaryl, C₅-C₂₀-Aryl; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch C₂-C₁₀-Aryle ersetzt sein, wobei die vorstehend genannten Aryle mit einem oder mehreren nichtaromatischen Substituenten R³ substituiert sein können, und
- m, n: jeweils eine ganze Zahl 0, 1, 2 oder 3, bevorzugt 0 oder 1 ist,

noch Struktureinheiten der Formel (E2) 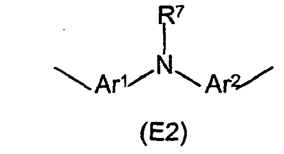
worin
- Ar¹, Ar²: mono- oder polycyclische aromatische konjugierte Systeme mit 2 bis 40 Kohlenstoffatomen sind, bei denen ein oder mehrere Kohlenstoffatome durch Stickstoff, Sauerstoff oder Schwefel ersetzt sein können, und welche mit einem oder mehreren Substituenten R³ substituiert sein können. Es ist dabei durchaus möglich bzw. teilweise sogar bevorzugt, daß die Aromaten Ar¹ und Ar² durch eine Bindung oder ein weiteres substituiertes oder unsubstituiertes C-Atom bzw. Heteroatom miteinander verknüpft sind und somit einen gemeinsamen Ring bilden.
- R⁷: gleich oder verschieden C₁-C₂₂-Alkyl, C₂-C₂₀-Heteroaryle oder C₅-C₂₀-Aryl; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein, wobei die vorstehend genannten Aryle/Heteroaryle mit einem oder mehreren nichtaromatischen Substituenten R³ substituiert sein können,
enthalten.

Ganz besonders bevorzugt werden die Struktureinheiten der Formel (E2) von folgenden Grundkörpem abgeleitet:
- Diphenylaminderivate, die in 4,4'-Stellung ins Polymer eingebaut sind;
- Phenothiazin- bzw. Phenoxazinderivate, die in 3,7-Stellung ins Polymer eingebaut sind;
- Carbazolderivate, die in 3,6-Stellung ins Polymer eingebaut sind;
- Dihydrophenazinderivate, die in 2,6- bzw. 2,7-Stellung ins Polymer eingebaut sind;
- Dihydroaciridinderivate, die in 3,7-Stellung ins Polymer eingebaut sind.

A2.2) Ebenso bevorzugt sind dabei Strukturen gemäß DE-A-19846766, die im folgenden aufgeführt sind. Diese Polymere enthalten Struktureinheiten gemäß Formel (F) 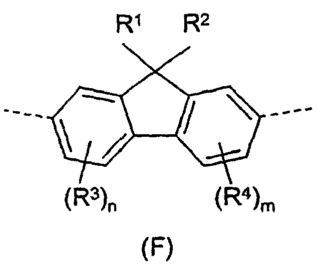
worin
- R¹, R²: zwei verschiedene Substituenten aus der Gruppe C₂-C₄₀-Aryl bzw. Heteroaryl darstellen; wobei die vorstehend genannten Aryle bzw. Heteroaryle mit einem oder mehreren Substituenten R³ substituiert sein können; verschiedenartig sollen die Aryle bzw. Heteroaryle im Sinne dieser Erfindung bereits sein, wenn sie sich durch die Art oder Stellung von Substituenten unterscheiden,
- R³, R⁴: gleich oder verschieden C₁-C₂₂-Alkyl, C₂-C₂₀-Aryl, F, Cl, CN, SO₃R⁵, NR⁵R⁶; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein können, wobei die vorstehend genannten Aryle mit einem oder mehreren nichtaromatischen Substituenten R³ substituiert sein können,
- R⁵, R⁶: gleich oder verschieden H, C₁-C₂₂-Alkyl, C₂-C₂₀-Aryl; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein, wobei die vorstehend genannten Aryle mit einem oder mehreren nichtaromatischen Substituenten R³ substituiert sein können, und
- m, n: jeweils eine ganze Zahl 0, 1, 2 oder 3, bevorzugt 0 oder 1 ist.

Ganz besonders bevorzugt stehen R¹, R² für zwei verschiedene Substituenten aus der Gruppe C₅-C₄₀-Aryl, C₂C₄₀-Heteroaryl; wobei die vorstehend genannten Aryle bzw. Heteroaryle mit einem oder mehreren Substituenten R³ substituiert sein können.

A2.3) Ebenso bevorzugt sind dabei Strukturen gemäß DE 19846768.0, die im folgenden aufgeführt sind. Dies sind Polyfluorene, die neben Einheiten gemäß Formel (E1) 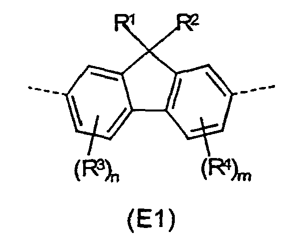
worin
- R¹, R²: gleich oder verschieden Wasserstoff, C₁-C₂₂-Alkyl, C₂-C₂₀-Aryl bzw. -Heteroaryl, F, Cl, CN; wobei die vorstehend genannten Alkylreste verzweigt oder unverzweigt sein können oder auch Cycloalkyle darstellen, und einzelne, nicht benachbarte CH₂-Gruppen des Alkyrestes durch O, S, C=O, COO, N-R⁵ oder auch einfache Aryle ersetzt sein können, wobei die vorstehend genannten Aryle mit einem oder mehreren Substituenten R³ substituiert sein können. Bevorzugt sind Verbindungen, bei denen R¹ und R² beide gleich und dabei ungleich Wasserstoff oder Chlor sind. Weiterhin bevorzugt sind Verbindungen, bei denen R¹ und R² voneinander verschieden und auch von Wasserstoff verschieden sind;
- R³, R⁴: gleich oder verschieden C₁-C₂₂-Alkyl, C₂-C₂₀-Aryl bzw. -Heteroaryl, F, Cl, CN, SO₃R⁵, NR⁵R⁶; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein können, wobei die vorstehend genannten Aryle mit einem oder mehreren nichtaromatischen Substituenten R³ substituiert sein können,
- R⁵, R⁶: gleich oder verschieden H, C₁-C₂₂-Alkyl, C₂-C₂₀-Aryl; dabei können die Alkylreste verzweigt oder unverzweigt sein oder auch Cycloalkyle darstellen; und einzelne, nicht benachbarte CH₂-Gruppen des Alkylrestes durch O, S, C=O, COO, N-R⁵ oder auch einfache Aryle ersetzt sein, wobei die vorstehend genannten Aryle mit einem oder mehreren nichtaromatischen Substituenten R³ substituiert sein können, und
- m, n: jeweils eine ganze Zahl 0, 1, 2 oder 3, bevorzugt 0 oder 1 ist,
auf jeden Fall noch Struktureinheiten gemäß Formel (G1) 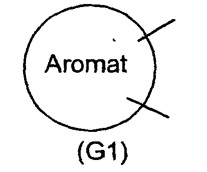 enthalten, worin
- Ar(omat): ein mono- oder polycyclisches aromatisches konjugiertes System mit 5bis 20 Kohlenstoffatomen ist, bei dem ein oder mehrere Kohlenstoffatome durch Stickstoff, Sauerstoff oder Schwefel ersetzt sein können, und dessen Verknüpfungsstellen so gewählt sind, daß sich entlang der Polymerhauptkette ein Winkel ungleich 180°, vorzugsweise kleiner 120°, insbesondere bevorzugt kleiner 90° ergibt.

Besonders bevorzugt sind hier Polymere enthaltend mindestens 1 Mol-%, vorzugsweise 2 Mol% bis 50 Mol-%, an Struktureinheiten (eine oder mehrere verschiedene) der Struktureinheit (G1).
Die Herstellung derartiger Polymere ist in DE-A-19846767, DE-A-19846766 und DE-A-19846768 eingehend beschrieben. Die Herstellung entsprechender erfindungsgemäßer Polymere kann durch das einpolymerisieren entsprechender Monomere, die die Oxetangruppierung tragen, erfolgen.

A3) Die Homo- und Copolymere des Poly-Spiros enthalten ein oder mehrere Struktureinheiten der Formel (H), wobei mindestens ein H-Atom im Polymer durch einen Substituenten gemäß Formel (A) ersetzt sind.

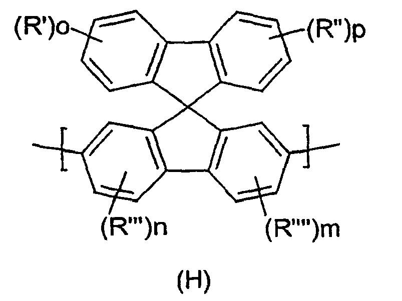

Die Substituenten R' bis R"" sind dabei gleich oder verschieden H, CN, F, Cl oder eine geradkettige, verzweigte oder cyclische Alkyl- oder Alkoxygruppe mit 1 bis 20 C-Atomen, wobei ein oder mehrere nicht benachbarte CH₂-Gruppen durch -O-, -S-, - CO-, -COO-, -O-CO-, -NR¹-, -(NR²R³)⁺-A⁻, oder -CONR⁴- ersetzt sein können und wobei ein oder mehrere H-Atome durch F ersetzt sein können, oder eine Arylgruppe mit 4 bis 40C-Atome, die durch einen oder mehrere, nicht aromatische Reste R' substituiert sein kann.
- R¹,R²,R³,R⁴: gleich oder verschieden aliphatische oder aromatische Kohlenwasserstoffreste mit 1 bis 20 C-Atomen oder auch H.
- A⁻:: ein einfach geladenes Anion oder dessen Äquivalent;
- n, m, o, p: 0, 1, 2, oder 3, bevorzugt 0,1 oder 2.

Bevorzugte Ausführungsformen der Poly-Spiros sind in (US-A-5621131) enthalten.

Die Herstellung derartiger Polymere ist in US-A-5621131 eingehend beschrieben. Die Herstellung entsprechender erfindungsgemäßer Polymere kann durch das einpolymerisieren entsprechender Monomere, die die Oxetangruppierung tragen, erfolgen.

B) Die niedermolekularen Verbindungen mit 3-dimensionaler Spirobifluorenstruktur bestehen aus Struktureinheiten der Formel (l1) 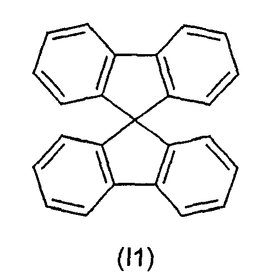
wobei die Benzogruppen unabhängig voneinander substituiert und/oder anelliert sein können und wobei mindestens ein H-Atom durch einen Substituenten gemäß Formel (A) ersetzt ist.

Bevorzugt werden hier Verbindungen gemäß EP-A-0676461 verwendet, wie diese in Formel (l2) wiedergegeben sind, 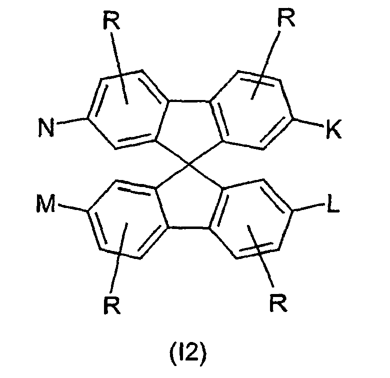
wobei die Symbole und Indizes folgende Bedeutungen haben:
K, L, M, N sind gleich oder verschieden 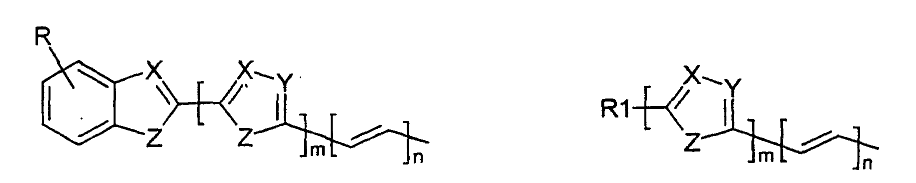 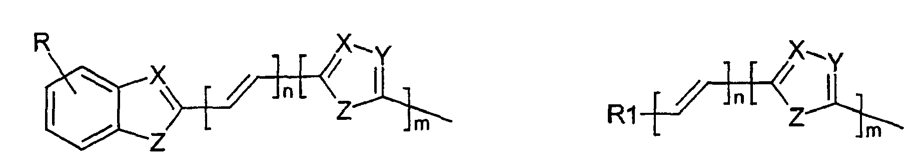 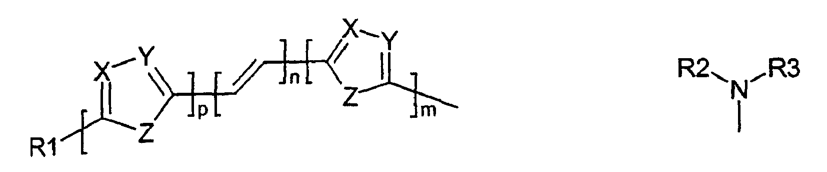
- R: kann, gleich oder verschieden, die gleichen Bedeutungen wie K, L, M, N haben oder ist -H, eine lineare oder verzweigte Alkyl, Alkoxy oder Estergruppe mit 1 bis 22 C-Atomen, -CN, -NO₂, -NR²R³, -Ar oder -O-Ar;
- Ar: ist Phenyl, Biphenyl, 1-Naphthyl, 2-Naphthyl, 2-Thienyl, 2-Furanyl, wobei jede dieser Gruppen einen oder zwei Reste R tragen kann,
- m, n, p: sind 0, 1, 2 oder 3;
- X, Y: sind gleich oder verschieden CR, N;
- Z: ist -O-, -S-, -NR¹-, -CR¹R⁴-, -CH=CH-, -CH=N-;
- R¹, R⁴: können, gleich oder verschieden, die gleichen Bedeutungen wie R haben
- R², R³: sind gleich oder verschieden H, eine lineare oder verzweigte Alkylgruppe mit 1 bis 22 C-Atomen, -Ar, 3-Methylphenyl.

Die Herstellung derartiger Verbindungen ist in EP 676461 eingehend beschrieben. Die Herstellung entsprechender erfindungsgemäßer Verbindungen kann durch das Ersetzen entsprechender Substituenten oder H-Atome durch die Oxetangruppierung gemäß Formel (A) erfolgen.

C) Die niedermolekularen Verbindungen mit 3-dimensionaler Triptycenstruktur bestehen aus Struktureinheiten der Formel (J) 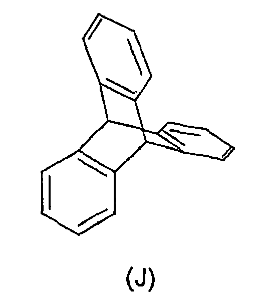
wobei die Benzogruppen unabhängig voneinander substituiert und/oder anelliert sein können und wobei mindestens ein H-Atom durch einen Substituenten gemäß Formel (A) ersetzt ist.

Bevorzugt werden hier Verbindungen gemäß DE-A-19744792 verwendet.

Die Herstellung derartiger Verbindungen ist in DE-A-19744792 eingehend beschrieben. Die Herstellung entsprechender erfindungsgemäßer Verbindungen kann durch das Ersetzen entsprechender Substituenten oder H-Atome durch die Oxetangruppierung gemäß Formel (A) erfolgen.

D) Die niedermolekularen Verbindungen mit 2-dimensionaler Triphenylenstruktur bestehen aus Struktureinheiten der Formel (K) 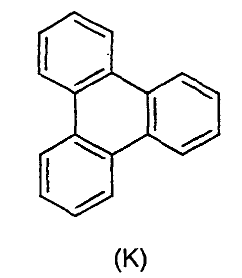
wobei die Benzogruppen unabhängig voneinander substituiert und/oder anelliert sein können wobei mindestens ein H-Atom durch einen Substituenten gemäß Formel (A) ersetzt ist.

Bevorzugt werden hier Verbindungen gemäß DE-A-4422332 verwendet.
Die Herstellung derartiger Verbindungen ist in DE-A-4422332 eingehend beschrieben. Die Herstellung entsprechender erfindungsgemäßer Verbindungen kann durch das Ersetzen entsprechender Substituenten oder H-Atome durch die Oxetangruppierung gemäß Formel (A) erfolgen.

E) Die Derivate des Perylen-tetracarbonsäure-diimids bestehen aus Struktureinheiten der Formel (L) 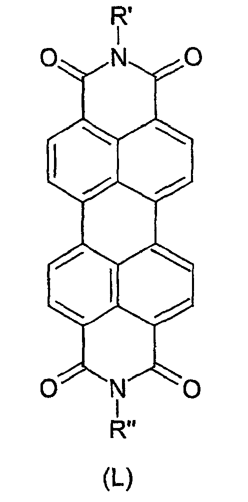
wobei die Benzogruppen unabhängig voneinander substituiert sein können und wobei mindestens ein H-Atom durch einen Substituenten gemäß Formel (A) ersetzt ist.
Diese Substituenten können dabei analog zu R', R" gleich oder verschieden eine geradkettige, verzweigte oder cyclische Alkyl- oder Alkoxygruppe mit 1 bis 20 C-Atomen, wobei ein oder mehrere nicht benachbarte CH₂-Gruppen durch -O-, -S-, - CO-, -COO-, -O-CO-, -NR¹-, -(NR²R³)⁺-A⁻, oder-CONR⁴- ersetzt sein können und wobei ein oder mehrere H-Atome durch F ersetzt sein können, oder eine Arylgruppe mit 4 bis 14 C-Atome, die durch einen oder mehrere, nicht aromatische Reste R' substituiert sein kann. Des weiteren können die von R' und R" verschiedenen Substituenten auch noch CN, F und Cl bedeuten.

Die Herstellung entsprechender erfindungsgemäßer Verbindungen kann durch das Ersetzen entsprechender Substituenten oder H-Atome durch die Oxetangruppierung gemäß Formel (A) erfolgen.

F) Die Derivate des Chinaciridon bestehen aus Struktureinheiten der Formel (M) 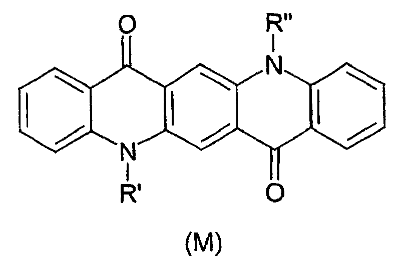
wobei die Benzogruppen unabhängig voneinander substituiert sein können und wobei mindestens ein H-Atom durch einen Substituenten gemäß Formel (A) ersetzt ist.
Die Substituenten können dabei analog zu R', R" gleich oder verschieden eine geradkettige, verzweigte oder cyclische Alkyl- oder Alkoxygruppe mit 1 bis 20 C-Atomen, wobei ein oder mehrere nicht benachbarte CH₂-Gruppen durch -O-, -S-, - CO-, -COO-, -O-CO-, -NR¹-, -(NR²R³)⁺-A⁻, oder -CONR⁴- ersetzt sein können und wobei ein oder mehrere H-Atome durch F ersetzt sein können, oder eine Arylgruppe mit 4 bis 14 C-Atome, die durch einen oder mehrere, nicht aromatische Reste R' substituiert sein kann. Desweiteren können die von R' und R" verschiedenen Substituenten auch noch CN, F und Cl bedeuten.

Die Herstellung entsprechender erfindungsgemäßer Verbindungen kann durch das Ersetzen entsprechender Substituenten oder H-Atome durch die Oxetangruppierung gemäß Formel (A) erfolgen.

G) Die organischen Lanthanoiden-Komplexe bestehen aus Struktureinheiten der Formel (N)

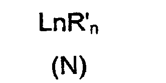

Die Substituenten R' können gleich oder verschieden Carboxylaten, Ketonaten, 1,3-Diketonaten, Imiden, Amiden, Alkoholaten sein, wobei mindestens ein H-Atom durch einen Substituenten gemäß Formel (A) ersetzt ist.
Die Anzahl der Liganden hängt vom jeweiligen Metall ab. Bevorzugt sind hier die organischen Komplexe des Europiums, Gadoliniums und des Terbiums, besonders bevorzugt diejenigen des Europiums.

Die Herstellung entsprechender erfindungsgemäßer Verbindungen kann durch das Ersetzen entsprechender Substituenten oder H-Atome in den Substituenten durch die Oxetangruppierung der Formel (A) erfolgen.

H) Die Derivate des Metall-chinoxalinats bestehen aus Struktureinheiten der Formel (O) 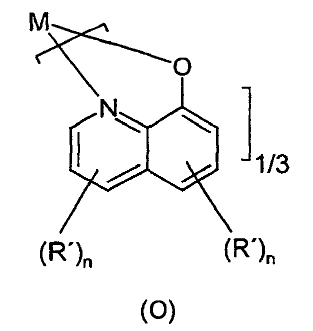
wobei die Benzogruppen unabhängig voneinander mit Resten R' substituiert sein können,
M steht für Aluminium, Zink, Gallium, Indium, bevorzugt Aluminium; n steht für eine ganze Zahl 0, 1, 2 oder 3.
Die Substituenten der Benzogruppe R' können dabei gleich oder verschieden eine geradkettige, verzweigte oder cyclische Alkyl- oder Alkoxygruppe mit 1 bis 20 C-Atomen, wobei ein oder mehrere nicht benachbarte CH₂-Gruppen durch -O-, -S-, - CO-, -COO-, -O-CO-, -NR¹-, -(NR²R³)⁺-A⁻, oder-CONR⁴- ersetzt sein können und wobei ein oder mehrere H-Atome durch F ersetzt sein können, oder eine Arylgruppe mit 4 bis 14 C-Atome, die durch einen oder mehrere, nicht aromatische Reste R' substituiert sein kann. Desweiteren können die von R' und R" verschiedenen Substituenten auch noch CN, F und Cl bedeuten.
Die erfindungsgemäße Oxetangruppe gemäß Formel (A) kann nun entweder ein H-Atom an einem der Chinoxalinringe ersetzen, oder auch an einem anderen Liganden R' sitzen, der einen der Chinoxalin-Liganden ersetzt.

I) Die Derivate des Oxadiazols bestehen aus Struktureinheiten der Formel (P) 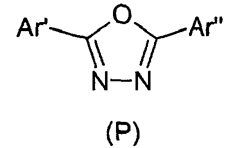
wobei Ar' und Ar" gleich oder verschieden einem substituierten oder unsubstituiertem Aromaten oder Heteroaromaten mit 4 bis 14 C-Atomen sein kann wobei mindestens ein H-Atom durch einen Substituenten gemäß Formel (A) ersetzt ist.

Bevorzugt ist Ar' und Ar" gleich oder verschieden Phenyl, 1- bzw. 2-Naphthyl, 1-, 2- bzw. 9-Anthracenyl, 2-, 3- bzw. 4-Pyridinyl, 2-, 4- bzw. 5-Pyrimidinyl, 2-Pyrazinyl, 3- bzw. 4-Pyridazinyl, 2-, 3-, 4-, 5-, 6-, 7- bzw. 8-Chinolin, 2- bzw. 3-Thiophenyl, 2- bzw. 3-Pyrrolyl, 2- bzw. 3-Furanyl.
Die möglichen Substituenten sind gleich oder verschieden, CN, F, Cl, CF₃ oder eine geradkettige, cyclische oder verzweigte Alkyl- oder Alkoxygruppe mit 1 bis 12 C-Atomen, wobei ein oder mehrere nicht benachbarte CH₂-Gruppen durch -O-, -S-, - CO-, -COO-, -O-CO-, -NR¹-, -(NR²R³)⁺-A-, oder -CONR⁴- ersetzt sein können und wobei ein oder mehrere H-Atome durch F ersetzt sein können.

Die erfindungsgemäße Oxetangruppe gemäß Formel (A) kann nun entweder ein H-Atom an einem der Arylringe ersetzen, oder auch an einem der Substituenten der Arylringe sitzen.

J) Polymere (Polystyrole) die Tetraaryl-benzidin-einheiten in der Seitenkette tragen, bestehen aus Struktureinheiten der Formel (Q) bzw. analogen Verbindungen bei anderen Polymergrundgerüsten (Polyacrylate, Polyamide, Polyester) 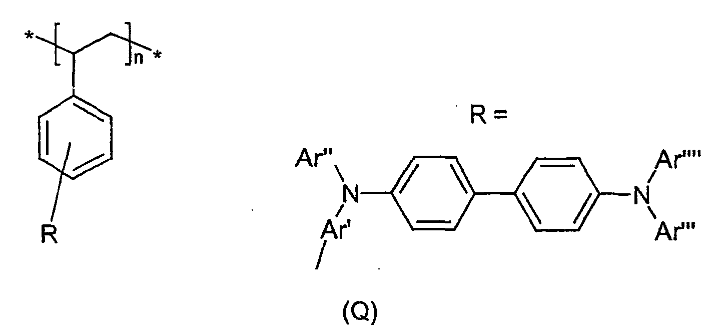
wobei Ar', Ar", Ar"' und Ar"" gleich oder verschieden einem substituierten oder unsubstituiertem Aromaten oder Heteroaromaten mit 4 bis 14 C-Atomen sein kann

Bevorzugt ist Ar', Ar", Ar"' und Ar"" gleich oder verschieden Phenyl, 1- bzw. 2-Naphthyl, 1-, 2- bzw. 9-Anthracenyl, 2-, 3- bzw. 4-Pyridinyl, 2-, 4- bzw. 5-Pyrimidinyl, 2-Pyrazinyl, 3- bzw. 4-Pyridazinyl, 2-, 3-, 4-, 5-, 6-, 7- bzw. 8-Chinolin, 2- bzw. 3-Thiophenyl, 2- bzw. 3-Pyrrolyl, 2- bzw. 3-Furanyl.

Die möglichen Substituenten sind gleich oder verschieden, CN, F, Cl, CF₃ oder eine geradkettige, cyclische oder verzweigte Alkyl- oder Alkoxygruppe mit 1 bis 12 C-Atomen, wobei ein oder mehrere nicht benachbarte CH₂-Gruppen durch -O-, -S-, - CO-, -COO-, -O-CO-, -NR¹-, -(NR²R³)⁺-A⁻, oder -CONR⁴- ersetzt sein können und wobei ein oder mehrere H-Atome durch F ersetzt sein können.

Diese Tetraaryl-Benzidin-Gruppe ist nun über einen Spacer, bevorzugt eine C1 bis C6 Alkyl-, Alkoxy- oder Estergruppierung mit der Polymer-Hauptkette verbunden.

Die erfindungsgemäße Oxetangruppe gemäß Formel (A) kann nun entweder ein H-Atom an einem der Arylringe ersetzen, oder an einem der Substituenten der Arylringe sitzen, oder auch an einem weiteren einpolymerisierten Monomer, welches keine Tetraaryl-Benzidin-Einheit trägt.

Die oben geschilderten Substanzen können nun als Reinstoff oder auch in Mischung untereinander bzw. auch mit anderen Hilfsstoffen verwendet werden.

Ein weiterer Gegenstand der Erfindung sind Abmischungen oder Formulierung enthaltend die erfindungsgemäßen Materialien und zugesetzte Hilfsstoffe, wie Initiatoren, und gegebenenfalls Sensibilisatoren, Stabilisatoren, Retarder, Inhibitoren, Reaktiv-Verdünner etc.

Den erfindungsgemäßen Materialien werden mindestens ein Photoinitiator oder ein Photo-Initiator-System zugemischt. Die Konzentration wird typischerweise im Bereich 0.1 bis 1.0 Gew.-% bezogen auf die erfindungsgemäße Verbindung/Polymer gewählt. Durch Bestrahlen mit aktinischer Strahlung wird eine Säure erzeugt, die durch kationische, ringöffnende Polymerisation eine Vemetzungsreaktion auslöst.

Durch strukturierte Bestrahlung kann so ein Muster von Bereichen mit vemetztem und Bereichen mit unvemetztem Material erhalten werden. Durch geeignete Operationen (z. B. Spülen mit geeigneten Lösemitteln), können dann die Bereiche mit unvernetztem Material entfernt werden. Dies führt zu der gewünschten Strukturierung.

Insofern die Vernetzung größerer Flächen gewünscht ist, kann nach Filmbildung die Vemetzung durch Aufbringen einer Säure initiiert werden.

Ein weiterer vorteilhafter und erfindungsgemäßer Effekt ist, daß durch die Vemetzung die mechanische und thermische Stabilität der mit den erfindungsgemäßen Materialien erzeugten Schichten steigt. Dies führt dazu, daß die Vorrichtungen, welche erfindungsgemäße - vernetzte - Materialien enthalten, deutlich langlebiger unter entsprechenden Bedingungen (z. B. thermische Beanspruchung, mechanischer Streß) sind. Dieser Effekt ist ausgeprägter, je höher der Vernetzungsgrad ist.

Hierdurch ist die nachfolgende Aufbringung der verschiedenen Schichten nach erfolgter Vemetzung erfolgen. So kann beispielsweise eine bereits vernetzte Schicht als Substrat zur Abscheidung von weiteren Stoffen aus der flüssigen Phase erfolgen. Der hierdurch erzielte Schichtaufbau kann bei gezielter Auswahl von Materialien mit unterschiedlichen optischen Brechungsindices als Wellenleiter genutzt werden.

Vorzugsweise wird der Emitter aus der Lösung auf eine bereits vernetzte Schicht eines Lochleiters oder Elektronenleiters aufgebracht werden. Mit Hilfe der erfindungsgemäßen Materialien ist die Herstellung mehrschichtiger Devices, unter Verwendung von löslichen aktiven Materialien, möglich.

Die Strukturierung erfolgt - wie oben beschrieben - durch Bestrahlung. Dies ist ein Standardprozeß in der heutigen Elektronik-Industrie und kann beispielsweise durch Bestrahlung mit Lasern oder durch flächige Bestrahlung durch eine entsprechende Maske erfolgen. Diese Maske birgt, im Gegensatz zum oben nachteilig geschilderten, keine Gefahr der Belegung, da hier ja nur Strahlung und kein Materialfluß durch die Maske zu verzeichnen ist. Die laterale Positionierung ist im Falle des Lasers als Quelle entweder durch Bewegung des Substrats, der Lichtquelle selbst oder durch optische Hilfsmittel möglich. Ein mögliches Hilfsmittel zur Substrat-Bewegung ist ein XY-Tisch der durch Schrittmotoren angetrieben wird. Optische Hilfsmittel können Spiegel sein. Besonders einfach und schnell ist die Strukturierung mittels Schattenmaske, die mit einer flächigen Lichtquelle, z.B. einer Hg-Höchstdrucklampe bestrahlt wird.

Ein weiterer Gegenstand der Erfindung sind die Schichten, die durch Vemetzung entstehen.

Gegenstand der Erfindung ist somit ebenfalls eine strukturierte organische Elektronik-Vorrichtung, im Sinne dieser Erfindung, mit einer oder mehreren aktiven Schichten, wobei mindestens eine dieser aktiven Schichten aus einem oder mehreren erfindungsgemäßen Materialen der Formel (A) hergestellt wurde.

Der allgemeine Aufbau für derartige Vorrichtungen ist oben beschrieben, im einzelnen sei hier nochmals aufgeführt:
(i) OLEDs und PLEDs analog EP 676 461 und WO 98-27136 und der darin zitierten Literatur.
(ii) Organische Solarzellen analog WO 94-05045 und WO 98-48433 und der darin zitierten Literatur.
(iii) Organische Laser analog WO 98-03566 und der darin zitierten Literatur.
(iv) Organische Schaltkreise (ICs) analog WO 95-31833 und WO 99-10939 und der darin zitierten Literatur.

Die Erfindung wird durch die nachfolgenden Beispiele näher erläutert, ohne sie dadurch einschränken zu wollen.

Teil A: Synthese der Monomere: Monomere für Einheiten gemäß Formel (D) (Fluorene)

### Beispiel M1: Herstellung von 9-(2,5-Dimethylphenyl)-9-(4-oxymethylen{2-[(3-ethylyloxetan)-3-yl) }phenyl)-2,7-dibromfluoren (M1)

In einem 50 ml Vierhalskolben wurden 808 mg (6 mmol) 2-Ethyl-2-chloromethyloxetan (siehe Chemphyschem. 2000, 207), 345 mg (5.2 mmol) KOH (85%ig) 2.60 g (5 mmol) 9-(2,5-Dimethylphenyl)-9-(4-hydroxyphenyl)-2,7-dibromfluoren (Siehe WO 00/22026) vorsichtig mit KOH versetzt und auf 150°C erhitzt. Dann wurden weitere drei Portionen von je 808 mg (je 6 mmol) 2-Ethyl-2-chloromethyloxetan über zwei Stunden zugegeben und das Gemisch bei dieser Temperatur noch weitere 2 h nachgerührt. Die Reaktion wurde durch DC (Hexan/CHCl₃ 1: 1) verfolgt.
Die Reaktionsmischung wurde abgekühlt und bei Raumtemperatur mit 50 ml Wasser versetzt. Dann wurde mit Essigester (50 ml) versetzt. Es fiel ein weißer Feststoff aus, der sich weder in der wässrigen noch in der organischen Phase löste. Er wurde abgesaugt. Man erhielt nach Trocknen im Vakuum 2.2 g (3.55 mmol, 71%) des Monomere M1, welches It. ¹H NMR > 99%ig war.
¹H NMR (Benzol-d₆, 400 MHz): 7.72 (dd, 2 H, J = 2.0, 0.8, Fluoren H1/H8); 7.28 (dd, 2 H, J = 2.0, 7.8, Fluoren H3/H6); 7.21 (br. s, 1 H, H-6" Phenyl); 7.06 (d mit Fs., 4 H, J = 8.0 Hz, H-Phenyl); 6.88-6.80 (m, 2 H, H3", H4"); 6.47 (d mit Fs., 2 H, j, 7.7 Hz, Fluoren H4/H5), 4.35 (d, J = 7.2 Hz, 2 H Oxetan); 4.25 (d, J = 7.0 Hz, 2 H Oxetan); 3.55 (s, 2H, OCH₂); 2.01 (s, 3H, CH₃); 1.55 (q, 2H, J = 6.8 Hz, CH₂-Ethyl); darunter bei 1.54 (s, 3H, CH₃); 0.61 (t, J = 6.8 Hz, CH₃-Ethyl).

### Beispiel M2: Herstellung von 9-(2,5-Dimethylphenyl)-9-{6-[(3-methyloxetan)-3-yl)methoxy]-hexoxy}-2,7-dibromfluoren (Monomer M2)

Analog Beispiel M1 wurden 2.22 g (5 mmol) 9-(2,5-Dimethylpehnyl)-9-hydroxy-2,7-dibromfluoren (Siehe WO 00/22026) mit 2.65 g (2 eq) (6'-Bromhexyloxy-3-methyl)-3-methyloxetan (siehe Polym. J. 1993; 25, 1283) bei 50°C für 23 h umgesetzt. Man erhielt 2.04 g (3.25 mmol, 65%) des Monomeren M2, welches It.¹H NMR > 98%ig war.
¹H NMR (CDCl₃, 400 MHz): 7.50 2 (br. s, 7 H, Fluoren-H, H6); 7.06 (d, 1 H, J = 8.0 Hz, H-3'); 6.88 (d, 1 H, J = 8.0 Hz, H-4'); 4.38 (d, J = 7.2 Hz, 2 H Oxetan); 4.22 (d, J = 7.0 Hz, 2 H Oxetan); 3.63 (m, 4H, OCH₂); 2.88 (s, 2H, OCH₂); 2.31 (s, 3H, CH₃); 1.78-1.20 (m, 14 H, 4 x CH₂, 2 x CH₃).

### Beispiel M3: Darstellung von 9,9-Bis{6-[(3-Methyloxetan)-3-yl)methoxy],hexoxy}-2,7-dibromfluoren (M3)

In einem 250 mL Einhalskolben mit Rückflusskühler wurden 1.44 g (60 mmol) NaH in 50 mL trockenem Toluol und 50 mL trockenem DMA suspendiert. Zu dieser Reaktionsmischung wurden 3.24 g (10 mmol) 2,7-Dibromfluoren und 5.57 g (21 mmol) (6'-Bromhexyloxy-3-methyl)-3-methyloxetan zugegeben und 1 h lang auf 100°C erhitzt. Nach Abkühlen auf Raumtemperatur wurde vorsichtig 1 mL H₂O mittels Pipette zugesetzt und dann 150 mL Et₂O zugegeben. Die organische Phase wurde mit 4 x 50 mL H₂O gewaschen, dann mit MgSO₄ getrocknet und die Lösungsmittel in vacuo entfernt. Das reine Produkt erhielt man durch Säulenchromatographie (2-fach) an Kieselgel mit einem Laufmittelgemisch aus Ethylacetat : Petrolether = 1 : 2. Ausbeute: 3.78 g (39%) viskoses Öl.
¹H-NMR (CDCl₃, 300 MHz): 0.65 (m, 4 H), 1.09 (m, 4 H), 1.27 (s, 6 H), 1.40 (m, 4 H), 1.89 (m, 4 H), 3.34 (t, J = 6.6 Hz, 4 H), 3.40 (s, 4 H), 4.31-4.47 (AA'BB'-System, J = 5.7 Hz, 8 H), 7.43-7.51 (AA'BB'-System, J = 8.1 Hz, 4 H), 7.43 (m, 2 H). 13C-NMR (CDCl3, 75 MHz): δ = 21.4 (CH3), 24.0 (CH2), 26.1 (CH2), 29.8 (CH2), 30.0 (CH2), 40.3 (C_{quart}), 40.5 (CH2), 56.0 (C_{quart}), 71.9 (CH₂), 76.4 (CH₂), 80.6 (CH2), 121.6 (CH), 121.9 (CH), 126.5 (CH), 130.6 (C_{quart}), 139.5 (C_{quart}.), 152.8 (C_{quart}). MS (70 eV, m/z (%)): 692 (M+, 100), 690 (50), 662 (12), 614 (12), 612(11), 349 (14), 323 (30), 245 (10), 243 (10), 85 (38), 55 (23). IR (Film, KBr): = 3048 cm⁻¹, 2931, 2860, 2797, 1598, 1570, 1450, 1416, 1398, 1378, 1264, 1117, 1061, 1004, 979, 940, 878, 835, 811, 741 666, 427. UV/vis (CHCl₃) λₘₐₓ (ε) = 282 nm (26178), 303 nm (14170), 314 nm (18116). C₃₅H₄₈Br₂O₄ (692.58): Ber.: C: 60.70, H: 6.99, Br: 23.07, Gef.: C: 61.24, H: 6.83, Br: 22.77.

### Beispiel M4: Darstellung von Monomer M4 (Siehe Schema 1)

Die Darstellung von M4 erfolgte Analog M1 mit 2.2 eq (6'-Bromhexyloxy-3-methyl)-3-methyloxetan. Man erhielt nach analoger Aufarbeitung 56% M4 als farblosen Feststoff.

Die Strukturen der verwendeten erfindungsgemäßen Monomere M1-M4 sowie weiterer Monomere sind in Schema 1 zusammengefaßt:

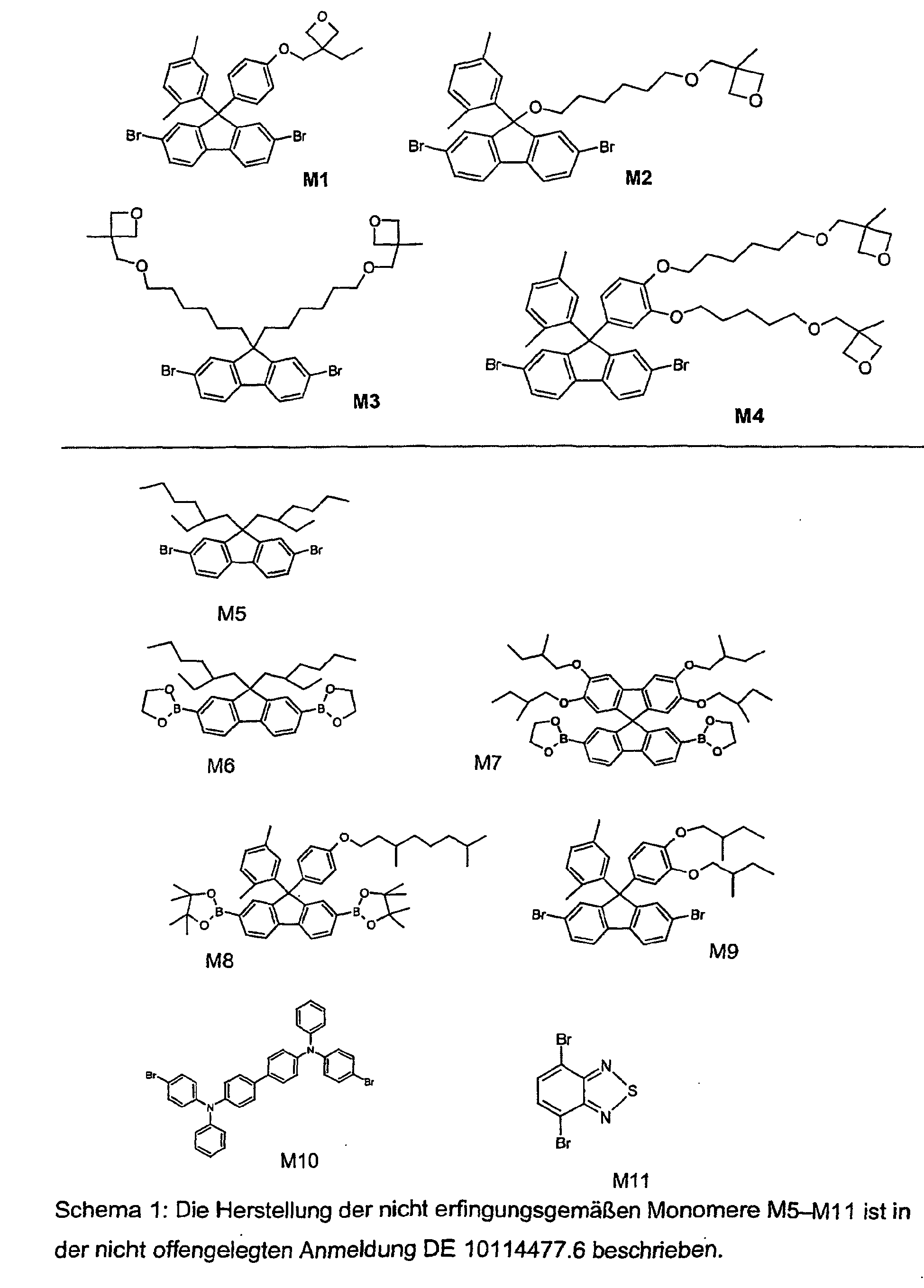

### Teil B: Herstellung der Polymere

### Beispiel P1: Copolymerisation von Monomer M1, 2,7-Dibrom-9-(2',5'-dimethylphenyl)-9-[3",4"-bis(2-methyl-butyloxy)phenyl]fluoren (M9), N,N'-Bis(4-bromophenyl)-N,N'-bisphenylbenzidin (M10) und 9-(3,7-dimethyloctyloxyphenyl)-9-(2,5-dimethylphenyl)fluoren-2,7-bisboronsäurepinacolylester (M8) durch Suzuki-Reaktion (Polymer P1).

1.6237 g (2.400 mmol) -2,7-Dibrom-9-(2',5'-dimethyl-phenyl)-9-[3",4"-bis(2-methyl butyloxy)phenyl]fluoren (M9), 4.5281 g (6.00 mmol) 9-(3,7-dimethyloctyloxyphenyl)-9-(2,5-dimethylphenyl)fluoren-2,7-bisboronsäurepinacoylester (M8), 0.7757g (1.200 mmol) N,N'-Bis(4-bromophenyl)-N,N'-bis(phenyl)benzidin (M10), 1.4842 g (2.400 mmol) Monomer M1, 5.80 g (25.2 mmol) K₃PO₄.H₂O, 18 ml Toluol, 9 ml Wasser und 0.15 ml Ethanol wurden 30 min durch Durchleiten von N₂ entgast. Anschließend wurde 57mg (0.05 mmol) Pd(PPh₃)₄ unter Schutzgas zugegeben. Die Suspension wurde unter N₂-Überlagerung bei 87°C Innentemperatur (leichter Rückfluß) kräftig gerührt. Nach 4 Tagen wurden weitere 0.10 g 9-(3,7-dimethyloctyloxyphenyl)-9-(2,5-dimethylphenyl)ftuoren-2,7-bisboronsäurepinacoylester zugesetzt. Nach weiteren 6 Stunden Erhitzen wurden 0.3 ml Brombenzol zugesetzt und noch 3 h zum Rückfluß erhitzt.
Die Reaktionslösung wurde mit 200 ml Toluol verdünnt, die Lösung wurde mit 200 ml 2%iger wäßriger NaCN 3h ausgerührt. Dabei hellte sich die Mischung nahezu vollständig auf. Die organische Phase wurde mit H₂O gewaschen und durch Zusetzen in 800 ml Ethanol gefällt. Das Polymer wurde in 200 ml Chloroform 1 h bei 40°C gelöst, über Celite filtriert und mit 250 ml MeOH ausgefällt, gewaschen und im Vakuum getrocknet. In 200 ml THF/ 250 ml Methanol wurde ein weiteres Mal umgefällt, abgesaugt und bis zur Massenkonstanz getrocknet. Man erhielt 4.80 g (9.64 mmol, 80 %) des Polymeren P1 als leicht gelben Feststoff.
¹H NMR (CDCl₃): 7.9-6.6 (m, Fluoren-H, Phenyl-H); 4.52 u 4.43(2 x d, J ~ 8Hz, je 0.4 H, Oxetan H); 4.0-3.4 (3 x m, OCH₂), 2.20 (s, CH₂-Ethyl, arom-CH₃); 1.9-0.7 (m, Alkyl H).
GPC: THF; 1 ml/min, PLgel 10µm Mixed-B 2 x 300 x 7.5 mm², 35°C, Rl Detektion: Mw = 77000 g/mol, Mn = 32000 g/mol.

Weitere Polymere wurden analog den Beschreibungen für P1 dargestellt. Die chemischen Eigenschaften sind in der folgenden Tabelle zusammengefaßt. Es wurden auch einige Vergleichspolymere ohne Oxetaneinheiten dargestellt. Auch diese sind in der Tabelle mit aufgeführt. All diese Polymere wurden auch für einen Einsatz in PLEDs untersucht. Wie PLEDs dargestellt werden können, ist zum einen oben schon ausgeführt und wird detaillierter noch in Teil C beschrieben.
Auch die wichtigsten Device-Eigenschaften (Farbe, Effizienz) sind in der Tabelle mit aufgeführt. Im Falle der Polymere P1-P9 sind die chemischen und elektrooptischen Daten der unvernetzten Polymere gezeigt. Die Vemetzung ist ebenfalls in Teil C beschrieben.

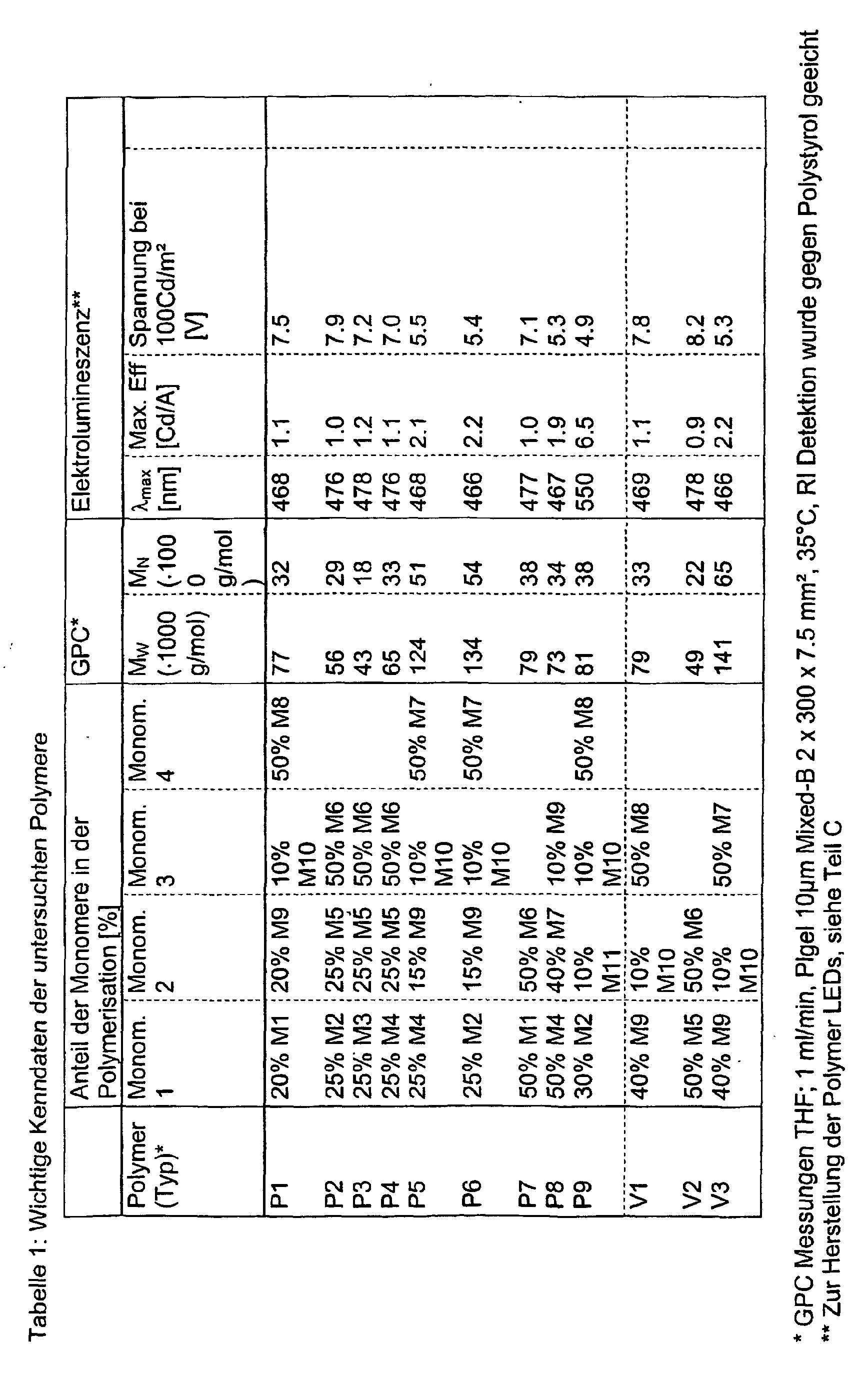

### Teil C: Herstellung und Charakterisierung von LEDs, Vernetzung der erfindungsgemäßen Polymere:

Die Herstellung von LEDs erfolgte nach dem im folgenden skizzierten allgemeinen Verfahren. Dieses mußte natürlich im Einzelfall auf die jeweiligen Gegebenheiten (z. B. Polymerviskosität und optimale Schichtdicke des Polymers im Device) angepaßt werden. Die im nachfolgenden beschriebenen LEDs waren jeweils Zweischichtsysteme, d. h. Substrat//ITO//PEDOT//Polymer//Kathode.
PEDOT ist ein Polythiophen-Derivat.

Allgemeines Verfahren zur Herstellung von hocheffizienten, langlebigen LEDs: Nachdem man die ITO-beschichteten Substrate (z. B. Glasträger, PET-Folie) auf die richtige Größe zugeschnitten hat, werden sie in mehreren Reinigungsschritten im Ultraschallbad gereinigt (z.B. Seifenlösung, Millipore-Wasser, Isopropanol). Zur Trocknung werden sie mit einer N₂-Pistole abgepustet und in einem Exsikkator gelagert. Vor der Beschichtung mit dem Polymer werden sie mit einem Ozon-Plasma-Gerät für ca. 20 Minuten behandelt. Von dem jeweiligen Polymer wird eine Lösung (in der Regel mit einer Konzentration von 4-25 mg/ml in beispielsweise Toluol, Chlorbenzol, Xylol:Cyclohexanon (4:1)) angesetzt und durch Rühren bei Raumtemperatur gelöst. Je nach Polymer kann es auch vorteilhaft sein, für einige Zeit bei 50 - 70°C zu rühren. Hat sich das Polymer vollständig gelöst, wird es durch einen 5µm Filter filtriert und bei variablen Geschwindigkeiten (400-6000) mit einem Spin-coater aufgeschleudert. Die Schichtdicken können dadurch im Bereich von ca. 50 und 300nm variiert werden. Vorab wird meist auf das (strukturierte) ITO ein leitfähiges Polymer, bevorzugt gedoptes PEDOT oder PANI aufgebracht.
Auf die Polymerfilme werden noch Elektroden aufgebracht. Dies geschieht in der Regel durch thermisches Verdampfen (Balzer BA360 bzw. Pfeiffer PL S 500). Anschließend wird die durchsichtige ITO-Elektrode als Anode und die Metallelektrode (z. B. Ba, Yb, Ca) als Kathode kontaktiert und die Device-Parameter bestimmt.
Die mit den beschriebenen Polymeren erhaltenen Resultate sind in der Tabelle in Teil B zusammengefaßt.

### Vernetzung:

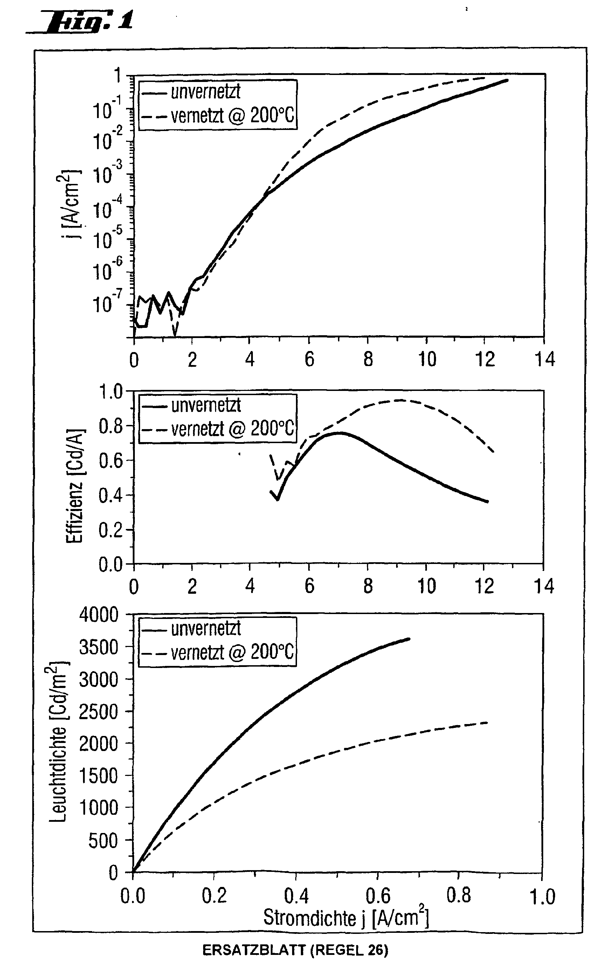

Die Vernetzung der erfindungsgemäßen Polymere wurde durch folgende Prozedur erreicht:
Die Polymere wurden in Toluol Lösung (1.5%ig) mit 3% gew.% (bezogen auf Polymer) Photosäure (4-(Thio-phenoxyphenyl)diphenylsulfonium hexafluoroantimonat) versetzt und wie oben beschrieben unter N₂ gespincoated. Nach Trocknen des Films wurde durch Bestrahlung mit einer UV Lampe (10 W, 302 nm, 5 Min) vernetzt. Der Film wurde dann unter N₂ 3 Minuten bei 200°C getempert (Beispiel P1, 130°C für Beispiele P2-P9) und dann mit einer 10⁻⁴ molaren LiAlH₄ Lösung in THF behandelt.
Die Device Daten für vemetztes und unvernetztes Polymer P1 sind in Figur 1 abgebildet. Die dabei gezeigten Werte wurden unter nicht optimierten Bedingungen (Kathode/Device Struktur) erhalten, sodaß die Absolutwerte der Effizienzen niedriger sind als in Tabelle 1.

### Nachweis der Vemetzbarkeit und der Photostrukturierbarkeit:

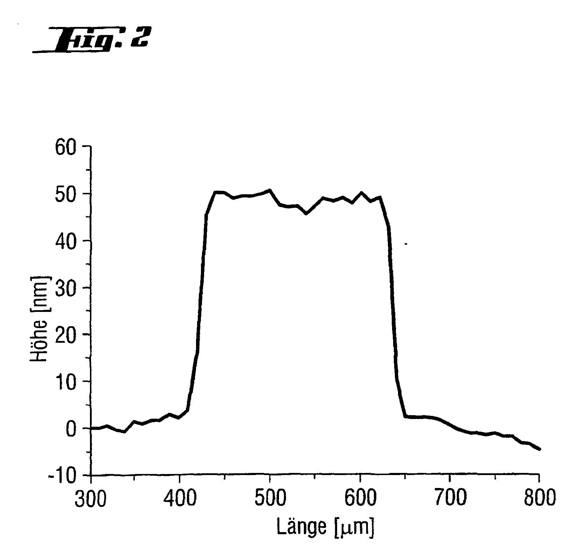

Es wurde aus dem mit Photosäure (s.o.) versetzten Polymer P5 ein 50 nm dünner Film hergestellt und durch eine Kammstruktur mit 200 µm Zinken für 20 sec. belichtet. Der Film wurde 20 Sekunden bei 120°C getempert und der lösliche Teil dann mit THF abgewaschen. Das mit einem Profilometer erhaltene Profil (Figur 2) zeigt gute Übereinstimmung mit der belichteten Struktur.

## Patentansprüche

1. Niedermolekulare oder polymere organische Elektrolumineszenz- bzw. Lasermaterialien zur Verwendung in elektronischen Anwendungen ausgewählt aus den Gruppen
A) Homo- oder Copolymere auf Basis von PPV oder Poly-Fluorenen oder Poly-Spiro,
B) Niedermolekulare Verbindungen mit 3-dimensionaler Spirobifluoren-Struktur,
C) Niedermolekulare Verbindungen mit 3-dimensionale Triptycen-Struktur,
D) Niedermolekulare Verbindungen mit 2-dimensionaler Triphenylen-Struktur,
E) Derivate des Perylen-tetra-carbonsäure-diimids,
F) Derivate des Chinaciridons,
G) organische Lanthanoid-Komplexe,
H) Derivate des Aluminium-tris-chinoxalinats,
I) Oxadiazol-Derivate
**dadurch gekennzeichnet, daß** diese mindestens eine zur Vemetzung befähigte Oxetan-Gruppe enthalten.

2. Materialien gemäß Anspruch 1 oder 2, **dadurch gekennzeichnet, daß** der Gehalt an Oxethangruppen definiert durch das molare Verhältnis von Oxetan-Ringen bezogen auf die Gesamtheit von organischen Ringen einschließlich der Oxetan-Ringe der jeweiligen Struktur zwischen 0,01 bis 0,6 beträgt.

3. Niedermolekulare oder polymere organische Elektrolumineszenz- bzw. Lasermaterialien gemäβ Anspruch 1 zur Verwendung in elektronischen Anwendungen ausgewählt aus den Gruppen
A) Homo- oder Copolymere auf Basis von PPV oder Poly-Fluorenen oder Poly-Spiro.
B) Niedermolekulare Verbindungen mit 3-dimensionaler Spirobifluoren-Struktur,
C) Niedermolekulare Verbindungen mit 3-dimensionale Triptycen-Struktur,
D) Niedermolekulare Verbindungen mit 2-dimensionaler Triphenylen-Struktur,
E) Derivate des Perylen-tetra-carbonsäure-diimids,
F) Derivate des Chinaciridons,
G) organische Lanthanoid-Komplexe,
H) Derivate des Aluminium-tris-chinoxalinats,
I) Oxadiazol-Derivate
**dadurch gekennzeichnet, daß** mindestens ein H-Atom durch eine Gruppe der Formel (A) ersetzt ist, 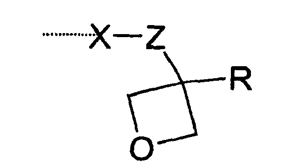
(A)
worin
R eine geradkettige, verzweigte oder cyclische Alkyl-, Alkoxyalkyl, Alkoxy- oder Thioalkoxy-Gruppe mit 1 bis 20 C-Atomen, C4-C18-Aryl, C2-C10-Alkenyt bedeuten bei denen ein oder mehrere Wasserstoffe durch Halogen, wie Cl und F, oder CN ersetzt sein können und ein oder mehrere nicht benachbarte C-Atome durch -O-, -S-, -CO-, -COO-, -O-CO-, ersetzt sein können,
Z für -O-, -S-, -CO-, -COO-, -O-CO- oder eine bivalente Gruppe -(CR₁R₂)ₙ- bei der R1 und R2 unabhängig voneinander Wasserstoff , eine geradkettige, verzweigte oder cyclische Alkyl-, Alkoxy-, Alkoxyalkyl oder Thioalkoxy-Gruppe mit 1 bis 20 C-Atomen, C4-C18-Aryl, C2-C10-Alkenyl bedeuten bei denen ein oder mehrere Wasserstoffe durch Halogen, wie Cl und F oder CN ersetzt sein können und ein oder mehrere nicht benachbarte C-Atome durch -O-, -S-, -CO-, -COO-, -O-CO- ersetzt sein können,
X eine bivalente Gruppe -(CR₁R₂)ₙ- bei der R1 und R2 unabhängig voneinander Wasserstoff , eine geradkettige, verzweigte oder cyclische Alkyl-, Alkoxy-, Alkoxyalkyl oder Thioalkoxy-Gruppe mit 1 bis 20 C-Atomen, C4-C18-Aryl, C2-C₁₀-Alkenyl bedeuten bei denen ein oder mehrere Wasserstoffe durch Halogen, wie Cl und F oder CN ersetzt sein können, und
n eine ganze Zahl zwischen 1 und 20, vorzugsweise zwischen 3 und 10, insbesondere 3 oder 6, bedeutet,
mit der Maßgabe, daß die Anzahl dieser Gruppen A durch die maximal verfügbaren, d.h. substituierbaren H-Atome begrenzt ist.

4. Materialien gemäß Anspruch 3, **dadurch gekennzeichnet, daß** der Gehalt an Oxethangruppe gemäß Formel (A) durch das molare Verhältnis von Oxetan-Ringen bezogen auf die Gesamtheit von organischen Ringen, einschließlich der Oxetan-Ringe in der jeweiligen Struktur zwischen 0,01 bis 0,6 beträgt.

5. Verwendung der Materialien gemäß einem oder mehreren der Ansprüche 1 bis 4 zur Herstellung von strukturierten Leuchtdioden, Lasern, Solarzellen, Wellenleitern oder integrierten Schaltkreisen.

6. Strukturierte organische Elektronik-Vorrichtung, mit einer oder mehreren aktiven Schichten, wobei mindestens eine dieser aktiven Schichten aus einem oder mehreren der Materialien gemäß einem der Ansprüche 1 bis 4 hergestellt wurde.

7. Vorrichtung gemäß Anspruch 6, **dadurch gekennzeichnet, daß** es sich um organische Leuchtdioden, Laser, Wellenleiter, Solarzellen oder organische Schaltkreise handelt.

8. Vorrichtung gemäß Anspruch 6, **dadurch gekennzeichnet, daß** die aktiven Schichten unterschiedliche Brechungsindices aufweisen.

9. Verfahren zur Herstellung mehrschichtiger organischer Elektronik-Vorrichtung, mit zwei oder mehreren aktiven Schichten, wobei mindestens eine dieser aktiven Schichten aus einem oder mehreren der Materialien gemäß einem der Ansprüche 1 bis 4, durch Aufbringen einer diskreten Schicht des aktiven Materials und dessen nachfolgender Vernetzung, Aufbringen einer weiteren aktiven Schichten aus einem oder mehreren der Materialien gemäß einem der Ansprüche 1 bis 4 und nachfolgender Vemetzung, gegebenenfalls Wiederholung der vorstehenden Schritte zum Aufbau der gewünschten Anzahl von Schichten.

## Claims

1. A low molecular weight or polymeric organic electroluminescent or laser material for use in electronic applications selected from the groups
A) a homo- or copolymer based on PPV or polyfluorenes or polyspiro
B) a low molecular weight compound having a 3-dimensional spirobifluorene structure,
C) a low molecular weight compound having a 3-dimensional triptycene structure
D) a low molecular weight compound having a 2-dimensional triphenylene structure
E) a derivative of perylenetetracarboxylic diimide
F) a derivative of quinaciridone
G) an organic lanthanide complex
H) a derivative of aluminum tris-quinoxalinate
I) an oxadiazole derivative
**characterized in that** at least one hydrogen atom is replaced by a group of the formula (A) 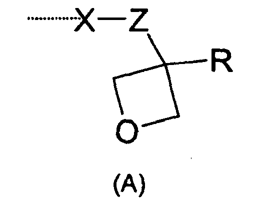
where
R is a straight-chain, branched or cyclic alkyl, alkoxyalkyl, alkoxy or thioalkoxy group having from 1 to 20 carbon atoms, C₄-C₁₈-aryl or C₂C₁₀-alkenyl, in each of which one or more hydrogen atoms may be replaced by halogen, such as Cl or F, or CN, and one or more nonadjacent carbon atoms may be replaced by -O-, -S-, -CO-, -COO- or -O-CO-,
Z is -O-, -S-, -CO-, -COO-, -O-CO- or a bivalent group -(CR₁R₂)ₙ- in which R₁ and R₂ are each independently hydrogen, a straight-chain, branched or cyclic alkyl, alkoxy, alkoxyalkyl or thioalkoxy group having from 1 to 20 carbon atoms, C₄-C₁₈-aryl or C₂-C₁₀-alkenyl, in each of which one or more hydrogen atoms may be replaced by halogen, such as Cl or F, or CN, and one or more nonadjacent carbon atoms may be replaced by -O-, -S-, -CO-, -COO- or -O-CO-,
X is a bivalent group -(CR₁R₂)ₙ- in which R₁ and R₂ are each independently hydrogen, a straight-chain, branched or cyclic alkyl, alkoxy, alkoxyalkyl or thioalkoxy group having from 1 to 20 carbon atoms, C₄-C₁₈-aryl or C₂-C₁₀₋alkenyl, in each of which one or more hydrogen atoms may be replaced by halogen, such as Cl or F, or CN, and
n is an integer from 1 to 20, preferably from 3 to 10, in particular 3 or 6,
with the proviso that the number of these A groups is limited by the maximum number of available, i.e. substitutable, hydrogen atoms.

2. A low molecular weight or polymeric organic electron conductor material for use in electronic applications, selected from the groups
A) a derivative of aluminum tris-quinoxalinate
B) an oxadiazole derivative.
**characterized in that** at least one hydrogen atom is replaced by a group of the formula (A) 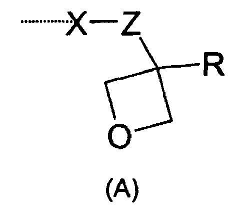
where
R is a straight-chain, branched or cyclic alkyl, alkoxyalkyl, alkoxy or thioalkoxy group having from 1 to 20 carbon atoms, C₄-C₁₈-aryl or C₂-C₁₀-alkenyl, in each of which one or more hydrogen atoms may be replaced by halogen, such as Cl or F, or CN, and one or more nonadjacent carbon atoms may be replaced by -O-, -S-, -CO-, -COO- or -O-CO-,
Z is -O-, -S-, -CO-, -COO-, -O-CO- or a bivalent group -(CR₁R₂)ₙ- in which R₁ and R₂ are each independently hydrogen, a straight-chain, branched or cyclic alkyl, alkoxy, alkoxyalkyl or thioalkoxy group having from 1 to 20 carbon atoms, C₄-C₁₈-aryl or C₂-C₁₀-alkenyl, in each of which one or more hydrogen atoms may be replaced by halogen, such as Cl or F, or CN, and one or more nonadjacent carbon atoms may be replaced by -O-, -S-, -CO-, -COO- or -O-CO-,
X is a bivalent group -(CR₁R₂)ₙ- in which R₁ and R₂ are each independently hydrogen, a straight-chain, branched or cyclic alkyl, alkoxy, alkoxyalkyl or thioalkoxy group having from 1 to 20 carbon atoms, C₄-C₁₈-aryl or C₂-C₁₀₋alkenyl, in each of which one or more hydrogen atoms may be replaced by halogen, such as Cl or F, or CN, and
n is an integer from 1 to 20, preferably from 3 to 10, in particular 3 or 6,
with the proviso that the number of these A groups is limited by the maximum number of available, i.e. substitutable, hydrogen atoms.

3. A material as claimed in claim 1 or 2, **characterized in that** the content of oxethane group according to formula (A) by the molar ratio of oxetane rings, based on all organic rings including the oxetane rings, in the particular structure is from 0.01 to 0.6.

4. The use of materials as claimed in one or more of claims 1 to 3 for producing structured light emitting diodes, lasers, solar cells, waveguides or integrated circuits.

5. A structured organic electronic device having one or more active layers, at least one of which has been produced from one or more of the materials as claimed in one of claims 1 to 3.

6. A device as claimed in claim 5, **characterized in that** it is an organic light emitting diode, laser, waveguide, solar cell or an organic circuit.

7. A device as claimed in claim 5, **characterized in that** the active layers have different refractive indices.

8. A process for producing multilayered organic electronic device having two or more active layers, at least one of which [lacuna] of one or more of the materials as claimed in one of claims 1 to 3, by applying a discrete layer of the active material and subsequently crosslinking it, applying a further active layer of one or more of the materials as claimed in one of claims 1 to 3 and subsequently crosslinking it, and optionally repeating the abovementioned steps to build up the desired number of layers.

## Revendications

1. Matériaux organiques d'électroluminescence ou laser de faible masse moléculaire ou polymères destinés à être utilisés dans des applications électroniques choisis dans les groupes
A) homo- ou copolymères à base de PPV ou de poly-fluorènes ou de poly-spiro,
B) composés de faible masse moléculaire à structure de spirobifluorène tridimensionnelle,
C) composés de faible masse moléculaire à structure de triptycène tridimensionnelle,
D) composés de faible masse moléculaire à structure de triphénylène bidimensionnelle,
E) dérivés du diimide d'acide pérylène-tétracarboxylique,
F) dérivés de la quinaciridone,
G) complexes de lanthanoïdes organiques,
H) dérivés du tris-quinoxalinate d'aluminium,
I) dérivés d'oxadiazole
**caractérisés en ce que** ceux-ci contiennent au moins un groupe oxétane capable de réticulation.

2. Matériaux selon la revendication 1 ou 2 **caractérisés en ce que** la teneur en groupes oxéthane définie par le rapport molaire des cycles oxétane à la totalité des cycles organiques y compris les cycles oxétane de la structure correspondante est entre 0,01 et 0,6.

3. Matériaux organiques d'électroluminescence ou laser de faible masse moléculaire ou polymères selon la revendication 1 destinés à être utilisés dans des applications électroniques choisis dans les groupes
A) homo- ou copolymères à base de PPV ou de poly-fluorènes ou de poly-spiro,
B) composés de faible masse moléculaire à structure de spirobifluorène tridimensionnelle,
C) composés de faible masse moléculaire à structure de triptycène tridimensionnelle,
D) composés de faible masse moléculaire à structure de triphénylène bidimensionnelle,
E) dérivés du diimide d'acide pérylène-tétracarboxylique,
F) dérivés de la quinaciridone,
G) complexes de lanthanoïdes organiques,
H) dérivés du tris-quinoxalinate d'aluminium,
I) dérivés d'oxadiazole
**caractérisés en ce qu'**au moins un atome H est remplacé par un groupe de formule (A) 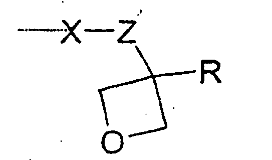
(A)
où
R représente un groupe alkyle, alcoxyalkyle, alcoxy ou thioalcoxy linéaire, ramifié
ou cyclique ayant 1 à 20 atomes C, C4-C18-aryle, C2-C10-alcényle où un ou plusieurs hydrogènes peuvent être remplacés par halogène, comme CI et F, ou CN et un ou plusieurs atomes C non voisins peuvent être remplacés par -O-, -S-, -CO-, -COO-, -O-CO-,
Z représente ―O-, -S-, -CO-, -COO-, -O-CO- ou un groupe divalent -(CR₁R₂)ₙ- où R1 et R2 représentent indépendamment l'un de l'autre l'hydrogène, un groupe alkyle, alcoxy, alcoxyalkyle ou thioalcoxy linéaire, ramifié où cyclique ayant 1 à 20 atomes C, C4-C18-aryle, C2-C10-alcényle où un ou plusieurs hydrogènes peuvent être remplacés par halogène, comme Cl et F ou CN et un ou plusieurs atomes C non voisins peuvent être remplacés par -O-, -S-, -CO-, -COO-, -O-CO-,
X représente un groupe divalent -(CR₁R₂)ₙ- où R1 et R2 représentent indépendamment l'un de l'autre l'hydrogène, un groupe alkyle, alcoxy, alcoxyalkyle ou thioalcoxy linéaire, ramifié ou cyclique ayant 1 à 20 atomes C, C4-C18-aryle, C2-C10-alcényle où un ou plusieurs hydrogènes peuvent être remplacés par halogène, comme Cl et F ou CN, et
n représente un nombre entier entre 1 et 20, de préférence entre 3 et 10, en particulier 3 ou 6,
avec la condition que le nombre de ces groupes A est limité par les atomes H disponibles au maximum, c'est-à-dire substituables.

4. Matériaux selon la revendication 3 **caractérisés en ce que** la teneur en groupe oxéthane selon la formule (A) par le rapport molaire des cycles oxétane à la totalité des cycles organiques, y compris les cycles oxétane dans la structure correspondante est entre 0,01 et 0,6.

5. Utilisation des matériaux selon une ou plusieurs des revendications 1 à 4 pour la production de diodes électroluminescentes, lasers, cellules solaires, guides d'ondes ou circuits intégrés structurés.

6. Dispositif électronique organique structuré, à une ou plusieurs couches actives, où au moins l'une de ces couches actives a été produite à partir d'un ou plusieurs des matériaux selon l'une des revendications 1 à 4.

7. Dispositif selon la revendication 6 **caractérisé en ce qu'**il s'agit de diodes électroluminescentes, lasers, guides d'ondes, cellules solaires organiques ou de circuits organiques.

8. Dispositif selon la revendication 6 **caractérisé en ce que** les couches actives présentent des indices de réfraction différents.

9. Procédé de production d'un dispositif électronique organique multicouche, à deux ou plusieurs couches actives, où au moins l'une de ces couches actives à partir d'un ou plusieurs des matériaux selon l'une des revendications 1 à 4, par application d'une couche discrète du matériau actif et sa réticulation subséquente, application d'une autre couche active à partir d'un ou plusieurs des matériaux selon l'une des revendications 1 à 4 et réticulation subséquente, éventuellement répétition des étapes précédentes pour la formation du nombre de couches souhaité.
